# EUROPEAN PATENT APPLICATION

(11) **EP 3 778 863 A2**
(43) Date of publication of application: **17.02.2021**
(21) Application number: 19778326.9
(22) Date of filing: 26.03.2019
(51) Int. Cl.: C12N 1/14, C12P 19/40, C12R 1/645

(54) **ADENOSINE-HIGH PRODUCTION PAECILOMYCES HEPIALI CS4 STRAIN ISOLATED FROM CORDYCEPS SINENSIS**

(30) Priority: 26.03.2018 KR 20180034770
(71) Applicant: LEE, Won Jae, Seoul 03043 (KR)
(72) Inventor: LEE, Won Jae, Seoul 03043 (KR)
(74) Representative: Hübner, Gerd
(86) International application number: PCT/KR2019/003485
(87) International publication number: WO 2019/190157

(57) **Abstract**

Provided is an adenosine-high production Paecilomyces hepiali Cs4 strain isolated from Cordyceps sinensis [accession number: KCTC 13285BP].

## Description

### [Technical Field]

The present invention relates to an adenosine-high production Paecilomyces hepiali Cs4 strain isolated from Cordyceps sinensis and, more particularly, to a Paecilomyces hepiali CS4 strain which is isolated from wild Cordyceps sinensis collected from Tibet region in China and has a remarkably enhanced capacity to produce adenosine.

### [Background Art]

In general, some of ascomycete molds grow in insects which serve as a host. When certain conditions are met, the molds break through the insects to create fruiting bodies which are collectively called cordyceps. Various cordyceps have been found depending on the types of insect as a host and the types of parasitic mold fungus.

As the cordyceps spp. fungus, it is known that there are hundreds of cordyceps species depending on strains and types of insect which serve as a host, such as Cordyceps militaris with silkworm pupa as a host, Paecilomyces japonica, Cordyceps scarabaeicola, Cordyceps mutans, Cordyceps sobolifera, Cordyceps sinensis, etc. Out of the species, Cordyceps sinensis, in which swift moth larva serves as a host, has been known to have the most excellent medical effect from a long time ago.

The Cordyceps sinensis is collected from the high mountains of Tibet and Chinghai regions in China, Nepal and like places, and is so valuable that Cordyceps sinensis has been treated as one of the three traditional medicinal herbs in China.

In China, however, the collection of Cordyceps sinensis is extremely restricted due to concerns about the destruction of natural ecology. As heavy metals such as arsenic have been recently found in natural cordyceps, there are growing concerns about the food safety of naturally collected cordyceps.

The Cordyceps sinensis is produced in such a way that sexual and asexual spores or hyphae infect a swift moth larva, and in winter, the hyphae take nutrients in the body of the swift moth larva and grow, and the larva dies. In summer, the Cordyceps sinensis fungus develops, and fruiting bodies grow from a head of a host, penetrate the host, and have the shape of a baseball stick.

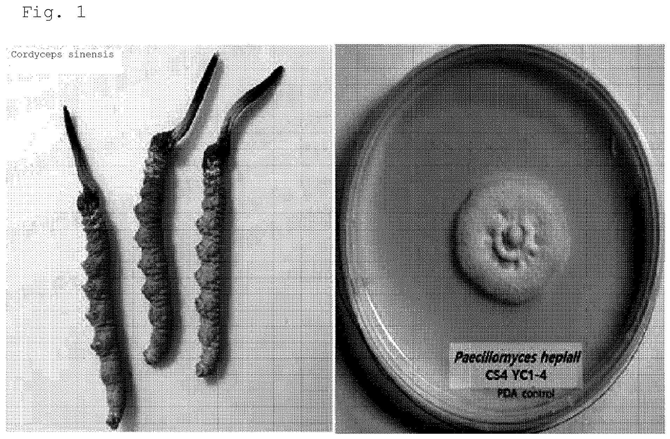

As shown in FIG. 1, the Cordyceps sinensis takes on a circular shape having a total length of about 10 cm and a diameter of about 0.5 cm. Initially, the Cordyceps sinensis is light green and turns into dark brown, and a head of the fruiting body is slightly bulging and has a cylindrical shape and an oval shape.

As the swift moth fungus, it is known that there are various fungi such as Opiocordyceps sinensis, Paecilomyces hepiali, Lecanicillium, etc.

Meanwhile, it is reported that the cordyceps contains various low-molecular substances such as polysaccharides, cordycepin, adenosine, lovastatin, ergosterol, various unsaturated fatty acids, etc., depending on types and thuscontributes to the physiological activity of the human body (Lo et al. A Systematic Review of the Mysterious Caterpillar Fungus Ophiocordyceps sinensis in Dong-ChongXiaCao (Cordyceps) and Related Bioactive Ingredients. J Tradit Complement Med, 2013, 3, 16-32) . Besides, it is known that the cordyceps is efficacious in hepatitis, diabetes, tuberculosis, etc., and has various effects on improving blood circulation as a tonic, strengthening immune functions, etc.

However, various species of cordyceps such as Cordyceps militaris, Paecilomyces japonica, etc., have been already successfully cultivated in an artificial way and have entered a stage of mass production. However, only Cordyceps sinensis, which is known to have the most excellent efficacy, has failed to be artificially cultivated until now, and thus is still dependent solely on natural production.

This is because there is a problem with technique for isolating seeds and technique for culturing the isolated seeds. Thus, it is necessary to develop the above two techniques so that the cordyceps can be artificially mass-produced, raw materials can be stably supplied for commercialization, and products can be standardized.

Meanwhile, Korean Registered Patent No. 10-0415893 discloses a "method for isolating Cordyceps sinensis fungus," and Korean Registered Patent No. 10-0459869 discloses a "method for culturing Cordyceps sinensis using a silkworm medium or a silkworm pupa medium" with regard to a method for culturing Cordyceps sinensis in a large quantity by using a silkworm or silkworm pupa as a host. However, there is no description about a Paecilomyces hepiali CS4 strain isolated from Codyceps synensis according to the present invention, and a method for culturing the same.

In addition, the Chinese Food and Drug Administration particularly recognizes adenosine as an index material for indicating the efficacy of cordyceps, but a culture method with an enhanced capacity to produce adenosine has not been described in the above-described related art.

Against this background, the present invention provides a culture method which isolates a Paecilomyces hepiali CS4 strain from Cordyceps sinensis collected from Tibet region in China, and has a remarkably enhanced capacity to produce adenosine, which is an index material for indicating the efficacy of cordyceps.

### [Disclosure]

### [Technical Problem]

The present invention is derived from the above needs, and an object of the present invention is to provide a Paecilomyces hepiali CS4 strain which is isolated from wild Cordyceps sinensis collected from Tibet region in China and has a remarkably enhanced capacity to produce adenosine.

### [Technical Solution]

To solve the above problem, the present invention provides a Paecilomyces hepiali CS4 strain [accession number: KCTC 13285BP], which is isolated from wild Cordyceps sinensis collected from Tibet region in China.

In the present invention, the strain may have an enhanced capacity to produce adenosine.

In the present invention, the strain may be cultured in a pupa medium.

### [Advantageous Effects]

According to an embodiment of the present invention, it may be possible to secure a Paecilomyces hepiali CS4 strain which has a remarkably enhanced capacity to produce adenosine, which is an index material for indicating an efficacy of cordyceps, compared to a wild strain.

In addition, it may be possible to produce adenosine with a high concentration and in a large quantity, in whichadenosine is an index material for indicating the efficacy of cordyceps and an ingredient effective in strengthening immunity, preventing aging, and improving the heart/liver.

According to the embodiments of the present invention, the effects are not limited to the content exemplified above, and more various effects are included in the present specification.

### [Description of Drawings]

FIG. 1 is a view showing a picture (left) of Cordyceps sinensis collected and a picture (right) in which a Paecilomyces hepiali CS4 strain isolated from Cordyceps sinensis is cultured in a culture plate.
FIG. 2 is an image (nos. 1 to 4 and 7 of isolated fungi, no. 5 of Codyceps militaris fungus, and no. 6 of Codyceps cicadae fungus) showing RAPD results for sorting Cordyceps sinensis molds according to the present invention.
FIG. 3 is a graph showing a comparison of adenosine production capacity among Cordyceps sinensis strains isolated according to the present invention.

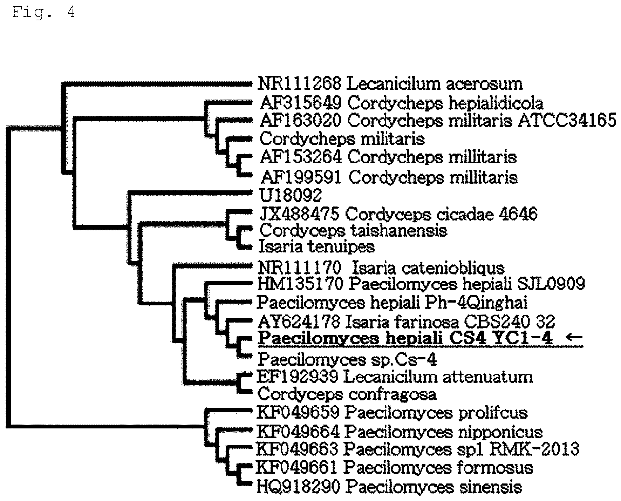

FIG. 4 is an image showing a comparison of ITS sequences among Cordyceps sinensis strains isolated.
FIG. 5 is a graph showing results of investigating an adenosine production capacity of Paecilomyces hepiali CS4 strain of the present invention with various materials as a medium.
FIG. 6 is a graph showing cross-breeding using haploid fungi derived from cordyceps spores.
FIG. 7 is a graph showing results of analyzing an adenosine content of Paecilomyces hepiali CS4 strains under each culture condition according to the present invention.

### [Best Mode]

Hereinafter, preferred embodiments of the present invention will be described in more detail with reference to the accompanying drawings.

According to one embodiment of the present invention, a Cordyceps sinensis strain is a strain belonging to Paecilomyces hepiali, and has been deposited to the Korea Research Institute of Bioscience and Biotechnology under the name of Paecilomyces hepiali YC1-4 as of June 13, 2017, and given the accession number KCTC 13285BP.

The above Paecilomyces hepiali YC1-4 strain may have an adenosine production capacity of 180 mg/100g medium when cultured in a pupa medium.

However, the culture of the strain is not limited to the types of medium, and may be also cultured in a brown rice medium, a brown rice + silkworm powder medium, etc.

The above-described adenosine is an index material for indicating an efficacy of cordyceps and is known to have a considerable efficacy on strengthening immunity, preventing aging, and improving the heart/liver.

The present invention may provide a cordyceps extract according to another embodiment.

The above cordyceps extract may be an extract of the Paecilomyces hepiali YC1-4 strain, contain adenosine as an effective ingredient, and further contain amino acids, vitamin precursors, mannitol and/or the like.

The above-described cordyceps extract may refer to not only a product obtained by extracting an effective ingredient from the Paecilomyces hepiali YC1-4 strain, but also a product obtained by purifying or processing the Paecilomyces hepiali YC1-4 strain per se or a part thereof.

In order to obtain the above-described cordyceps extract, it may be possible to use a conventional method for extracting an extract from a natural product in the art. Specifically, an extract of the Paecilomyces hepiali YC1-4 strain can be obtained according to a method of using a conventional extraction solvent under conventional temperature and pressure conditions. As an extraction solvent, it may be possible to use solvents generally used in an extraction process, such as water, anhydrous or water-containing lower alcohol having 1 to 4 carbon atoms, acetone, ethyl acetate, butyl acetate, dichloromethane, 1,3-butylene glycol, etc.

The present invention may provide health functional foods, health supplement foods, health foods, cosmetics, drug medicines, quasi-drugs and the like, which contain the cordyceps extract according to another embodiment.

The health functional foods, health supplement foods, health foods, cosmetics, drug medicines and quasi-drugs containing the above-described cordyceps extract may further contain ingredients which are sitologically, cosmetically or pharmaceutically acceptable.

The above-described sitologically acceptable ingredients may include, for example, proteins, carbohydrates, fats, nutrients, seasoning agents and the like. Specifically, the carbohydrates may include, in particular, monosaccharides (glucose, fructose, etc.), disaccharides (maltose, sucrose, etc.), oligosaccharides, polysaccharides (dextrin, cyclodextrin, etc.), and sugar alcohols (xylitol, sorbitol, erythritol, etc.), and natural flavoring agents (taumatin, stevia extract, etc.) and synthetic flavoring agents (saccharin, aspartame, etc.) as a flavoring agent.

The above-described cosmetically or pharmaceutically acceptable ingredients may include, for example, carriers, binders, glidants, disintegrants, excipients, solubilizers, stabilizers, bases, lubricants, preservatives, humectants, emulsifiers, suspending agents, viscosity controlling agents, antiseptics, surfactants, antioxidants, moisturizers, fragrances, pigments and the like.

In addition, the drug medicines and quasi drugs containing the above-described cordyceps extract may be formulated into a preparation by using a pharmaceutically acceptable carrier and/or excipient according to a method easily practicable by those skilled in the art to which the present invention pertains, and thus may be prepared in a unit dose form or prepared by being inserted into a multi-dose container.

In this case, a dosage form may be in a form of solution, suspension or emulsion in an oil or aqueous medium, or may be in a form of extract, powder, granule, tablet or capsule, and may further contain dispersing agents or stabilizing agents.

Hereinafter, experimental examples will be described in detail with regard to isolation of the above Paecilomyces hepiali YC1-4 strain, a culture method, and investigation of adenosine production capacity.

### Example 1: Isolation and culture of strains according to the present invention

### - Isolation of fungus strains from wild Cordyceps sinensis -

Wild Cordyceps sinensis, which was collected from Chinghai region in China as shown in FIG. 1, was washed with 70% ethanol, after which the washed cordyceps was cut and the resulting internal tissue was cut into pieces in a size of 0.1 to 0.3 cm, and cultured in a PDA medium (including ampicillin 100 mg/mℓ) at a temperature of 18°C for one week or longer.

Then, the fungi growing through a cultured period were transferred to a new PDA medium for pure culture, so that a total of five types of fungus were isolated therefrom.

### Example 2: Sorting of molds isolated from Cordyceps sinensis and investigation of adenosine production capacity

### Sorting of molds isolated from Cordyceps sinensis -

An experiment was conducted to confirm whether the isolated fungi are the same as or different from each other by using an RAPD method. For this purpose, chromosomal DNA was first extracted from the hyphae of each fungus, after which the extracted chromosomal DNA was isolated by using an i-genomic plant DNA extraction kit (Intron Biotech).

Then, 0.1 mM of OPS1 (CTACTGCGCT), OPS10 (GTCGTTCCTG) primers, 10 mℓ of 2X PCR premix (Intron Biotech) and 8 mℓ of distilled water were respectively added into 1 mℓ of mold DNA isolated as above, after which a PCR reaction was performed.

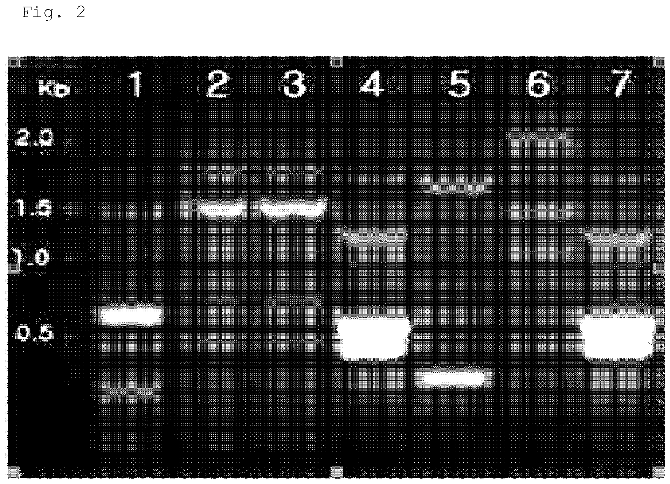

PCR was repeated 35 times under the condition of heating at 94°C for 10 minutes, heating at 94°C for 45 seconds, heating at 48°C for 30 seconds, and heating at 72°C for one minute, after which the PCR product was confirmed through electrophoresis with 1% agarose gel. As a result, it was confirmed that strain nos. 1 to 4 and 7 are isolated fungi, strain no. 5 is Codyceps militaris, and strain no. 6 is Codyceps cicadae, as shown in FIG.2, and it was judged that among the strains corresponding to the isolated fungi, the strain no. 2 and the strain no. 3 are the same fungi and the strain no. 4 and the strain no. 7 are the same fungi.

Thus, a total of three kinds of mold (strains nos. 1, 2 and 4) were isolated.

### - Investigation of adenosine production capacity of each isolated strain -

The adenosine production capacity of each strain isolated as above was investigated.

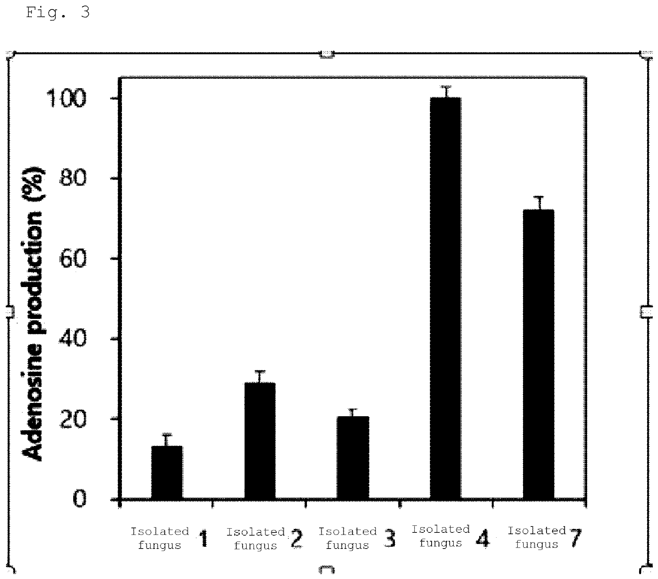

For this purpose, each of the isolated strains (nos. 1, 2 and 4) was cultured in a PDB medium for one week while being maintained at 20°C, after which a content of adenosine produced was compared. As shown in FIG. 3, among the isolated strains, the strain no. 4 had the highest content of adenosine, and the strain no. 7, which was identified as a strain of the same species by the RAPD, had the production capacity that corresponds to about 80% of the strain no. 4, and the remaining three strains showed a significant decrease in adenosine production capacity.

### Example 3: Identification of fungi isolated from cordyceps

Through the above experiment, an internal transcribed spacer (ITS) sequence was determined for the strain no. 4 among the fungus strains isolated from Cordyceps sinensis.

As a result of comparing the sequence determined in the strain no. 4 with the sequences of the existing cordyceps fungi, it was found that the strain isolated through the present invention is consistent with the Paecilomyces hepiali CS4 strain.

Accordingly, the above-described strain no. 4 was named as Paecilomyces hepiali CS4 YC1-4 according to the present invention.

### Example 4: Study on culture conditions of Paecilomyces hepiali CS4 YC1-4 according to the present invention

To figure out the optical culture conditions for Paecilomyces hepiali CS4 YC1-4 (hereinafter, **"YC1-4"**), which is the strain isolated from Cordyceps sinensis according to the present invention, the adenosine production capacity of YC1-4 strain obtained from the present invention was investigated in eight medium materials.

For this purpose, eight kinds of medium material such as soybeans, pupae, black beans 1, black beans 2, kidney beans, peanuts, cashew nuts, and rice were prepared, after which 50 g of each medium material was washed with water, and sterilized with the addition of 30 ml of water. The YC1-4 strain according to the present invention, which had been incubated in a PDB medium for one week, was inoculated in an amount of 3 mℓ into each of sterilized medium materials.

After inoculating the YC1-4 strain of the present invention into each of the above medium materials, the culture was carried out at 23°C for 20 days under a dark condition. After the culture was finished, a sample was collected under each of the culture conditions to analyze an adenosine content.

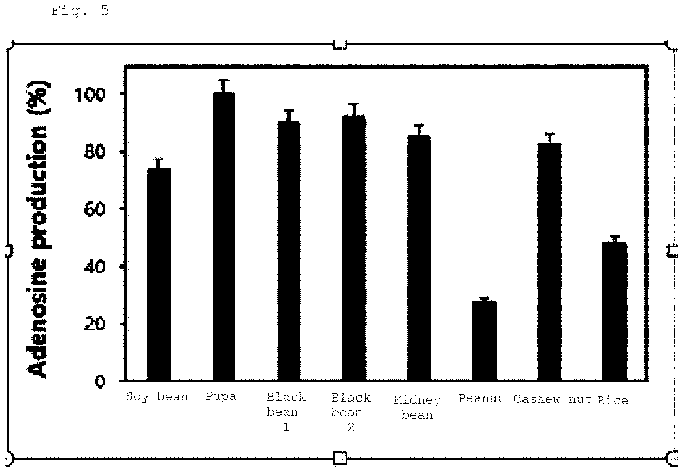

As a result, as shown in FIG. 5, a largest amount of adenosine was produced from the pupae among the eight medium materials.

Through the above experiment, the pupa was the most excellent medium material for the production of adenosine. Thus, the capacity to produce adenosine was investigated in the pupa medium as shown in FIG. 1, and was further investigated in brown rice and brown rice + silkworm powder media.

The medium was prepared by the same manner as above, and the YC1-4 fungi of the present invention were inoculated and incubated for 20 days under the condition of (1) light, (2) dark, (3) alternately light for 12 hours and dark for 12 hours, etc.

PupaeBrown riceBrown rice + Silkworm powder

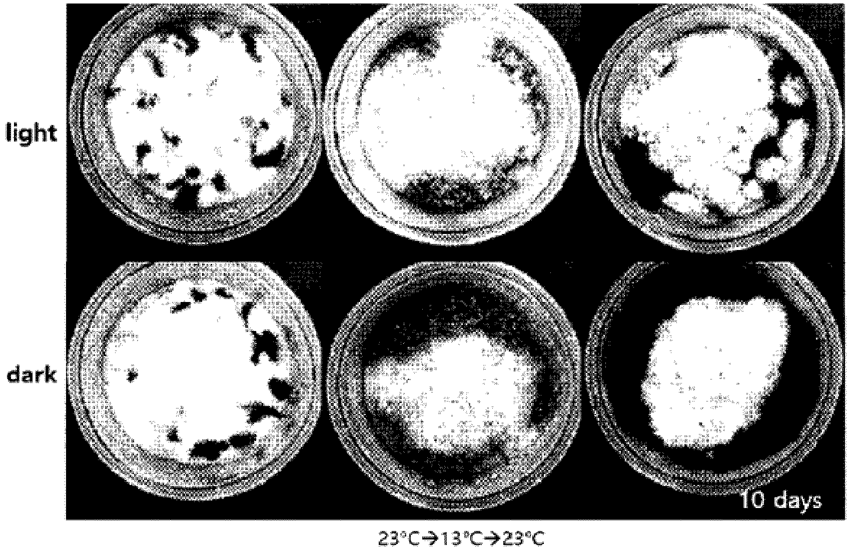
light
dark
10 days
23°C→13°C→23°C

FIG. 1. Shape of Paecilomyces hepiali CS4 YC1-4 (abbreviation: YC1-4) strain of the present invention after 10-day culture

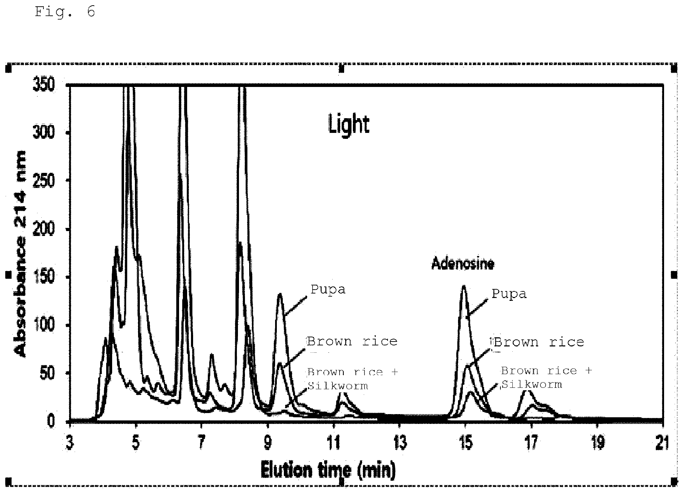

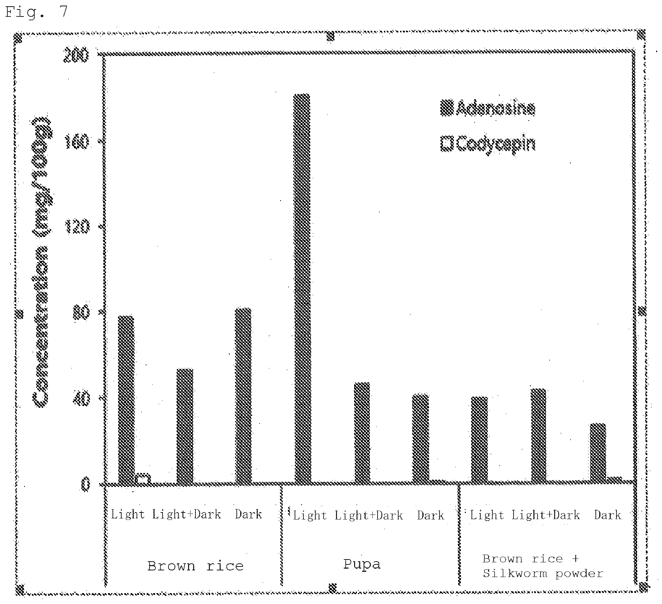

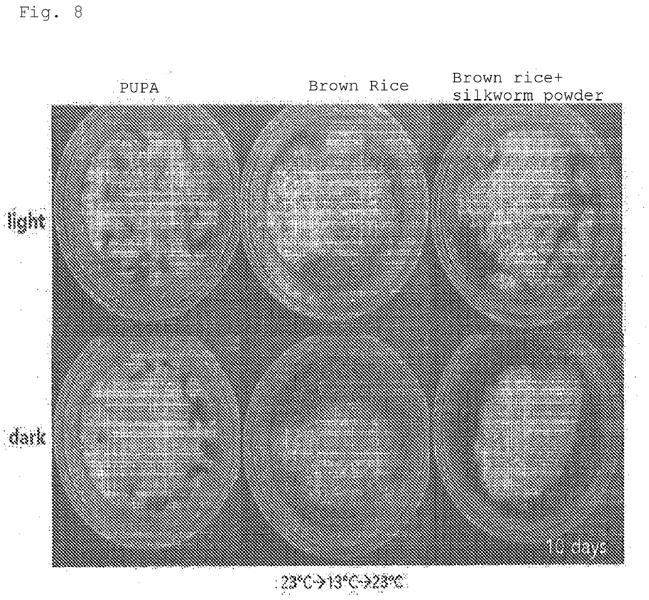

After the culture was completed as described above, a content of adenosine in each sample was analyzed by HPLC, and the analysis results and overall results were shown in FIGS. 6 and 7, respectively.

According to FIGS. 6 and 7, the highest capacity to produce adenosine was shown in the pupa medium incubated under the light condition, and an amount of adenosine production reached 180 mg/100 g medium under this condition.

The general adenosine production capacity of Cordyceps sinensis fungi, such as Paecilomyces hepiali CS4 or Opiocordyceps sinensis, is merely about 0.3-0.5 mg/g medium.

However, in the present invention, it is found that the adenosine production capacity of Paecilomyces hepiali CS4 YC1-4 strain, which is newly isolated through the optimization of medium conditions, reaches 180 mg/100 g medium. Thus, it can be confirmed that the adenosine production capacity thereof is remarkably enhanced compared to the existing Cordyceps sinensis strain.

- 1 -
Certificate of depositof microorganism
Budapest Treaty on the International Recognition of the Deposit of
Microorganisms for the Purposes of Patent Procedure
International Form
Receipt in the case of an original deposit

Issued pursuant to Article 7.1

To: Lee Won-Jae

Address: 18-2, Hyoja-ro 9-gil, Jongno-gu, Seoul, Korea

I. Identification of the microorganisms
Identification reference given by | Accession number given by
the depositor: | International Depository Authority:
Paecilomyces hepiali YC1-4 | KCTC 13285BP
II. Scientific description and/or proposed taxonomicdesignation
The microorganisms identified under I above was accompanied by:
[ ] a scientific description
[ ] a proposed taxonomicdesignation
(Mark with a cross where applicable)
III. Receipt and Acceptance
This International DepositoryAuthority accepts the microorganism under I
above, which was received by it on June 13, 2017.
IV. Receipt of request for conversion
The microorganism identified under I above was received by this
International DepositoryAuthority and a request to convert the original
deposit to a deposit under the Budapest Treatywas received by it on the
same date.
V. International DepositoryAuthority
Name: Korean Cell Line Bank | Signature(s) by person(s) having
Address:Korea Research Institute of | the power to represent the
Bioscience and Biotechnology (KRIBB) |

181, Ipsin-gil, Jeongeup- | International DepositoryAuthority
si,Jeollabuk-do,Korea | or of authorized official(s):
 | ----------------------
 | Director Kim Cha-Yeong
 | June 19, 2017

The content of the translated text is not different from that of the
original text.

## Claims

1. An adenosine-high production Paecilomyces hepiali CS4 strain isolated from Cordyceps sinensis [accession number: KCTC 13285BP], wherein 3 mℓ of YC1-4 strain, which is isolated from Cordyceps sinensis and incubated in a PDB medium for one week, is inoculated into 50 g of a pupa medium material, which is washed and sterilized with an addition of 30 mℓ of water, and a resulting product is incubated at 23°C under a light condition for 20 days, so that a yield of adenosine is 180 mg per 100 g of the medium.
